# EUROPÄISCHE PATENTANMELDUNG

(11) **EP 2 944 338 A1**
(43) Veröffentlichungstag der Anmeldung: **18.11.2015**
(21) Anmeldenummer: 14001678.3
(22) Anmeldetag: 13.05.2014
(51) Int. Cl.: A61M 1/10

(54) **Autonomes Herzkreislauf-Implantat mit einem Rückschlagventil**

(71) Anmelder: von Wattenwyl, Robert, 72124 Gniebel-Pliezhausen (DE)
(72) Erfinder: von Wattenwyl, Robert, 72124 Gniebel-Pliezhausen (DE)
(74) Vertreter: Schaad, Balass, Menzl & Partner AG

(57) **Zusammenfassung**

Das erfindungsgemässe autonome Herzkreislauf-Implantat (10A") hat einen mit einer Arterie (62) eines Herzkreislaufsystems (52) strömungsverbunden hochdruckseitigen Eingang (16) und einen mit einer Herzmuskelvene (66) strömungsverbunden niederdruckseitigen Ausgang (18). Ein Strömungspfad (46) ist vom hochdruckseitigen Eingang (16) zum niederdruckseitigen Ausgang (18) ausgebildet. Das Herzkreislauf-Implantat (10A") hat zudem einen vom hochdruckseitigen Eingang (16) weggehenden, stromaufwärts liegenden Leitungsabschnitt (20) des Strömungspfades (46) mit einem blasenartig ausdehnbaren Bereich (22) und einen zum niederdruckseitigen Ausgang (18) führenden, einen Strömungsquerschnitt (24) aufweisenden stromabwärts liegenden Leitungsabschnitt (26) des Strömungspfades (46). Der blasenartig ausdehnbare Bereich (22) ist elastisch zwischen einem erschlafften Zustand (30) und einem gedehnten Zustand (32) hin und her bewegbar. Entsprechend ist im erschlafften Zustand (30) der Strömungsquerschnitt (24) des stromabwärts liegenden Leitungsabschnitts (26) freigegeben und im gedehnten Zustand (32) ist, in vorliegendem Fall, der Strömungsquerschnitt (24) des stromabwärts liegenden Leitungsabschnitts (26) in einem Reduktionsbereich (25) vollständig reduziert. Erfindungsgemäss ist im Strömungspfad (46) ein Rückschlagventil (90, 90', 9C") angeordnet, welches die Strömung vom hochdruckseitigen Eingang (16) zum niederdruckseitigen Ausgang (18) zulässt und in Gegenrichtung unterbindet.

## Beschreibung

Die vorliegende Erfindung betrifft ein autonomes Herzkreislauf-Implantat mit einem Rückschlagventil gemäss dem Patentanspruch 1.

Die US 4,459,977 offenbart ein Herzkreislauf-Implantat mit einer pulsatilen Einheit zur diastolischen Retroperfusion des Koronarsinus synchron zur Frequenz des Herzschlags. Die pulsatile Einheit weist eine aufblasbare flexible Hülle auf, welche integral mit einem Leitungssystem ausgebildet ist. Die flexible Hülle ist an einem hochdruckseitigen Ende mit dem arteriellen System und an einem niederdruckseitigen Ende mit dem Venensystem verbunden. Weiter weist die pulsatile Einheit ein starres Gehäuse auf, in welchem die flexible Hülle angeordnet ist. Das starre Gehäuse ist mit einer Steuereinheit verbunden, um in einem Raum zwischen dem starren Gehäuse und der flexiblen Hülle - während der Diastole - ein Gas, wie z.B. Luft, einzubringen und - während der Systole - in diesem Raum ein Vakuum zu erzeugen. Beim Erzeugen des Vakuums - während der Systole - füllt sich die flexible Hülle mit arteriellem Blut und beim Einbringen von Gas in den Raum-während der Diastole - wird das arterielle Blut in Richtung des Venensystems, insbesondere zum Koronarsinus, für die retrograde Koronarperfusion abgeführt.

Die pulsatile Einheit des bekannten Herzkreislauf-Implantats funktioniert ausschliesslich mittels der ausserhalb des Körpers angeordneten Steuereinheit und kann ohne diese die gewünschte Wirkung der retrograden Koronarperfusion nicht erzielen. Die Steuereinheit muss insbesondere das Aufblasen und Entleeren der elastischen Hülle der pulsatilen Einheit aktiv unterstützen.

Die US 4,195,623 A offenbart eine zur Aorta parallel angeordnete Ballonpumpe als Herzkreislauf-Implantat zur Unterstützung des Herzkreislaufsystems. Die Ballonpumpe ist mit ihrem oberen Ende am aufsteigenden Teil der Aorta in unmittelbarer Nähe zum Herzen und mit ihrem entgegengesetzten Ende am thorakalen Teil der Aorta, ohne Unterbrechung des Blutflusses im Herzkreislaufsystem, verbunden. Die Ballonpumpe hat einen Innenschlauch und einen Aussenschlauch, wobei zwischen beiden Schläuchen ein aufblasbarer Ballon angeordnet ist. Der Ballon ist durch einen Verbindungsschlauch mit einer ausserhalb des Körpers angeordneten Kontrolleinheit verbunden. Die Kontrolleinheit ermöglicht das Aufblasen des Ballons mit Luft oder das Entleeren der Luft aus dem Ballon entgegengesetzt der Pulsation des Herzens. Entsprechend wird während der diastolischen Phase des Herzens der Ballon mittels der Kontrolleinheit mit Luft gefüllt, so dass das im Innenschlauch vorliegende Blut zurück in Richtung des Herzens gedrückt wird und während der systolischen Phase des Herzens ist die Ballonpumpe inaktiv, so dass die Kontrolleinheit die Luft wieder aktiv aus dem Ballon entleert.

Ein weiteres herzkreislaufunterstützendes Herzkreislauf-Implantat ist beispielsweise eine intraarterielle Ballonpumpe (IABP) wie sie in der WO 2011/039091 A1 offenbart ist. Beim IABP handelt es sich um ein mechanisches Herzunterstützungssystem, welches bei einem low cardic output-Syndrom und/oder bei einer sehr schlechten Koronardurchblutung zum Einsatz kommt. Über eine Arteria femoralis wird ein Katheter nach oben in die Aorta geschoben. Dieser Katheter hat einen länglichen Ballon. Die Platzierung erfolgt so, dass der Ballon im Bereich des Brustkorbs zu liegen kommt, unterhalb des Abgangs der linken Arteria subklavia und oberhalb des Abgangs der Nierenarterien. Ein ausserhalb des Körpers befindliches Steuergerät nimmt die Herzaktionen des Patienten mittels EKG (Elektrokardiogramm) oder arteriellen Druckkurven auf und füllt während der diastolischen Phase kurzzeitig den Ballon mit Helium. Am Ende der eingestellten Füllzeit wird das Gas im Ballon sehr schnell aktiv wieder abgesaugt, so dass der Ballon sich wieder entleert. Dies bewirkt, dass während der diastolischen Füllphase des Ballons das Blut nicht aus dem Bereich oberhalb des Ballons in die untere Körperhälfte abfliessen kann. Dadurch wird der diastolische Druck oberhalb des Ballons stark angehoben und somit auch der koronare Perfusionsdruck. Entsprechend kann die Versorgung des Herzmuskels mit sauerstoffreichem Blut auf diese Weise verbessert werden. Durch das schlagartige Entleeren des Ballons kurz vor der nächsten systolischen Phase fällt der Druck im Bereich der Aorta oberhalb des Ballons stark ab, weil das dort angestaute Blut jetzt frei in die untere Körperhälfte abfliessen kann.

Nachteilig an den oben bekannten mechanischen Herzkreislaufunterstützungssystemen ist die Anwendung einer Ballonpumpe unter Verwendung von Gasen, wie Helium oder Luft, zum aktiven Aufblasen und Entleeren des Ballons in der systolischen bzw. diastolischen Phase des Herzens. Zudem muss das Aufblasen und Entleeren des Ballons mit der Druckkurve des Herzens oder durch das EKG mittels einer externen Steuereinheit synchronisiert werden.

Es ist somit eine Aufgabe der vorliegenden Erfindung, ein Herzkreislauf-Implantat zur Herzkreislaufunterstützung bereitzustellen, welches autonom - ohne eine externe Energiequelle und ohne eine externe Steuereinheit-arbeitet.

Die Aufgabe wird mit einem autonomen Herzkreislauf-Implantat gelöst, welches die Merkmale des Patentanspruchs 1 aufweist.

Das erfindungsgemässe autonome Herzkreislauf-Implantat gemäss dem Patentanspruch 1 weist einen hochdruckseitigen Eingang auf, der dazu bestimmt ist, mit einer Arterie eines Herzkreislaufsystems strömungsverbunden zu sein, und weist einen niederdruckseitigen Ausgang auf, der dazu bestimmt ist, mit einer Vene, insbesondere einer Herzmuskelvene, des Herzkreislaufsystems strömungsverbunden zu sein. Entsprechend kann ein Strömungspfad vom hochdruckseitigen Eingang zum niederdruckseitigen Ausgang vorliegen. Zudem hat das Herzkreislauf-Implantat einen vom hochdruckseitigen Eingang weggehenden, stromaufwärts liegenden Leitungsabschnitt des Strömungspfades mit einem blasenartig ausdehnbaren Bereich und einen zum niederdruckseitigen Ausgang führenden, einen strömungsquerschnitt aufweisenden, stromabwärts liegenden Leitungsabschnitt des Strömungspfades. Der stromaufwärts liegende Leitungsabschnitt ist entweder mit seinem blasenartig ausdehnbaren Bereich ausserhalb jedoch neben dem stromabwärts liegenden Leitungsabschnitt oder im Inneren des stromabwärts liegenden Leitungsabschnitts angeordnet. Der blasenartig ausdehnbare Bereich ist elastisch zwischen einem erschlafften Zustand mit einem Ruhequerschnitt und einem gedehnten Zustand mit einem bezüglich des Ruhequerschnitts grösseren Arbeitsquerschnitt hin und her bewegbar. Auf diese Weise ist im erschlafften Zustand des blasenartig ausdehnbaren Bereichs der Strömungsquerschnitt des stromabwärts liegenden Leitungsabschnitts freigegeben und im gedehnten Zustand des blasenartig ausdehnbaren Bereichs ist der Strömungsquerschnitt des stromabwärts liegenden Leitungsabschnitts in einem Reduktionsbereich reduziert. Erfindungsgemäss ist im Strömungspfad ein Rückschlagventil angeordnet, welches die Strömung vom hochdruckseitigen Eingang zum niederdruckseitigen Ausgang zulässt und in Gegenrichtung unterbindet.

Auf diese Art und Weise kann - im implantierten Zustand des Herzkreislauf-Implantats - mittels des Rückschlagventils ein Rückfluss des Blutes vom Herzen in Richtung zum niederdruckseitigen Ausgang des Herzkreislauf-Implantats unterbunden werden.

Unter reduziert wird verstanden, dass der blasenartig ausdehnbare Bereich von seinem Ruhequerschnitt in den Arbeitsquerschnitt übergeht, jedoch diesen noch nicht vollständig erreicht haben muss, so dass der Strömungsquerschnitt zwar reduziert ist, jedoch nicht vollständig. Dieser Vorgang ist als zeitabhängiger Prozess zu verstehen, d.h. bei Verwendung einer pulsierenden Quelle füllt sich der blasenartig ausdehnbare Bereich wegen einer Druckwelle stetig mit Fluid, wodurch der gedehnte Zustand eingenommen wird und reduziert dabei stetig den Strömungsquerschnitt des stromabwärts liegenden Leitungsabschnitts.

Ein wesentlicher Vorteil bei der vorliegenden Erfindung liegt darin, dass auf externe Steuereinheiten und Füllmedien zum Aufblasen sowie Entleeren eines Ballons mit Gasen oder externen Flüssigkeiten vollständig verzichtet werden kann, damit dieses Herskreislauf-Implantat funktioniert. Entsprechend liegt ein völlig autonomes System vor.

Es sei an dieser Stelle erwähnt, dass es für den blasenartig ausdehnbaren Bereich von Vorteil ist, dass dieser sich beim Druckanstieg elastisch bis zum Arbeitsquerschnitt ausdehnen und beim Druckabfall wieder in seine ursprüngliche Form mit dem Ruhequerschnitt zusammenziehen kann.

Bei einer bevorzugten Ausführungsform des autonomen Herzkreislauf-Implantats ist das Rückschlagventil stromabwärts des Reduktionsbereichs angeordnet. Durch diese spezifische Anordnung des Rückschlagventils kann selbst bei reduziertem Leitungsquerschnitt des stromabwärts liegenden Leitungsabschnitts im Reduktionsbereich - im implantierten Zustand des Herzkreislauf-Implantats - das Blut immer nur vom hochdruckseitigen Eingang zum niederdruckseitigen Ausgang fliessen und nicht umgekehrt. Entsprechend ist mittels des Rückschlagventils eine Strömung des Blutes vom niederdruckseitigen Ausgang zum hochdruckseitigen Eingang des Herzkreislauf-Implantats vollständig unterbunden.

Bei einer bevorzugten Ausführungsform des autonomen Herzkreislauf-Implantats ist das Rückschlagventil zwischen dem blasenartig ausdehnbaren Bereich und dem Reduktionsbereich angeordnet. Diese spezifische Anordnung des Rückschlagventils hat den Vorteil, dass - im implantierten Zustand des Herzkreislauf-Implantats während der Systole - Blut vom blasenartig ausdehnbaren Bereich ausschliesslich in Richtung zum Reduktionsbereich abfliessen kann.

Es ist auch denkbar, dass in einer Ausführungsform des autonomen Herzkreislauf-Implantats das Rückschlagventil zwischen dem hochdruckseitigen Eingang und dem blasenartig ausdehnbaren Bereich angeordnet ist. Dies hätte zur Folge, dass - im implantierten Zustand des Herzkreislauf-Implantats während der Systole - kein arterielles Blut vom Herzen über den hochdruckseitigen Eingang wieder zurück in das Herzkreislaufsystem zurückfliessen kann.

Bei einer bevorzugten Ausführungsform des autonomen Herzkreislauf-Implantats ist bei vollständig gedehntem Zustand des blasenartig ausdehnbaren Bereichs der Strömungsquerschnitt des stromabwärts liegenden Leitungsabschnitts auf Null reduziert. Dies bedeutet einen vollständigen Unterbruch des Strömungspfades vom hochdruckseitigen Eingang zum niederdruckseitigen Ausgang sobald der blasenartig ausdehnbare Bereich seinen maximalen Arbeitsquerschnitt eingenommen hat.

Bei einer bevorzugten Ausführungsform des autonomen Herzkreislauf-Implantats ist bei der Anordnung des blasenartig ausdehnbaren Bereichs ausserhalb jedoch neben dem stromabwärts liegenden Leitungsabschnitt, dieser mit dem stromaufwärts liegenden Leitungsabschnitt durch einen u-förmigen Verbindungsleitungsabschnitt strömungsverbunden und die genannten Leitungsabschnitte sind in einem Gehäuse aufgenommen. Dies bewirkt bei Erreichen des maximalen Arbeitsquerschnitts des blasenartig ausdehnbaren Bereichs eine Wechselwirkung mit dem daneben angeordneten stromabwärts liegenden Leitungsabschnitt sowie dem Gehäuse. Hierbei hat das Gehäuse eine starre Wandung und wirkt in Form einer Gegenkraft der Ausdehnung des blasenartig ausdehnbaren Bereichs entgegen, so dass bei gedehntem Zustand der stromabwärts liegende Leitungsabschnitt zwischen dem Gehäuse und dem blasenartig ausdehnbaren Bereich gequetscht wird. Dies wiederum führt zum reduzierten Strömungsquerschnitt im stromabwärts liegenden Leitungsabschnitt.

Bei einer bevorzugten Ausführungsform des autonomen Herzkreislauf-Implantats ist bei der Anordnung des blasenartig ausdehnbaren Bereichs im Inneren des stromabwärts liegenden Leitungsabschnitts, letzterer als Gehäuse ausbildet. Auf diese Weise kann der blasenartig ausdehnbare Bereich im gedehnten Zustand mit dem als stromabwärts liegenden Leitungsabschnitt ausgebildeten, eine starre Wandung aufweisenden Gehäuse zusammenwirken. Hierbei verdrängt im gedehnten Zustand der blasenartig ausdehnbare Bereich das Raumvolumen des stromabwärts liegenden Leitungsabschnitts und verringert dabei den strömungsquerschnitt des stromabwärts liegenden Leitungsabschnitts vom hochdruckseitigen Eingang zum niederdruckseitigen Ausgang.

Bei einer bevorzugten Ausführungsform des autonomen Herzkreislauf-Implantats ist der Strömungspfad vom hochdruckseitigen Eingang zum niederdruckseitigen Ausgang abzweiglos ausgebildet. Dies bewirkt im erschlafften Zustand einen kontinuierlichen Strömungspfad vom hochdruckseitigen Eingang zum niederdruckseitigen Ausgang ohne Unterbruch.

Bei einer bevorzugten Ausführungsform des autonomen Herzkreislauf-Implantats ist der blasenartig ausdehnbare Bereich des stromaufwärts liegenden Leitungsabschnitts aus einem elastischen Material, insbesondere Elastomer, hergestellt. Dies bewirkt die benötigte Ausdehnung des blasenartig ausdehnbaren Bereichs im Verhältnis zu den restlichen Leitungsabschnitten zwischen dem hochdruckseitigen Eingang und niederdruckseitigen Ausgang.

Es ist insbesondere von Vorteil, wenn das Elastomer ein Form-Gedächtnis aufweist, so dass der blasenartig ausdehnbare Bereich im erschlafften Zustand und im gedehnten Zustand immer die gleiche gewünschte Form und wirkung aufweist.

Bei einer bevorzugten Ausführungsform des autonomen Herzkreislauf-Implantats sind sämtliche Leitungsabschnitte und Gehäuse aus biokompatiblem Material hergestellt. Dies ist insbesondere nach Implantation des Herzkreislauf-Implantats in den Körper von Bedeutung, damit das umliegende körpereigene Gewebe das Herzkreislauf-Implantat nicht als Fremdkörper abstösst.

Bei einer bevorzugten Ausführungsform des autonomen Herzkreislauf-Implantats ist die Innenwand der Leitungsabschnitte mit einer gerinnungsfreien Materialbeschichtung, insbesondere mit einer Heparinbeschichtung, versehen. Dies bewirkt eine Vermeidung einer Gerinnung aufgrund des Fremdkörperkontaktes, denn die Gerinnung würde zu einer Verstopfung der Leitungsabschnitte führen. Insbesondere durch die Beschichtung der Innenwand der Leitungsabschnitte mit der Heparinbeschichtung kann die Gerinnung vermieden werden.

Bei einer bevorzugten Ausführungsform des autonomen Herzkreislauf-Implantats weisen die verwendeten Materialien für die Leitungsabschnitte und das Gehäuse jeweils eine glatte Oberfläche auf, um einen laminaren Strömungspfad vom hochdruckseitigen Eingang zum niederdruckseitigen Ausgang zu gewährleisten.

Bei einer bevorzugten Ausführungsform des autonomen Herzkreislauf-Implantats ist der hochdruckseitige Eingang an einer grosskalibrigen Arterie, insbesondere einer Halsschlagader (Arteria carotis) oder einer Unterschlüsselbeinarterie (Arteria subclavia), als hochdruckseitiger Ausgang des Herzens anschliessbar.

Bei einer bevorzugten Ausführungsform des autonomen Herzkreislauf-Implantats ist der niederdruckseitige Ausgang an der Vene, insbesondere der Herzmuskelvene, zur Verbindung mit einem Koronarvenensinus (Sinus coronarius) als niederdruckseitigem Eingang des Herzens, anschliessbar.

Weitere Vorteile und Eigenschaften des erfindungsgemässen autonomen Herzkreislauf-Implantats gehen aus der nachstehenden Beschreibung von Ausführungsbeispielen hervor, welche anhand der Zeichnungen erläutert werden.

Es zeigen rein schematisch:

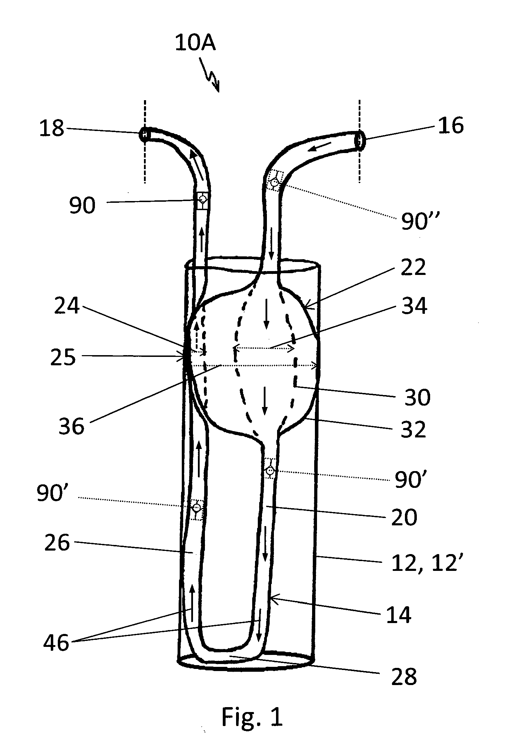

- Fig. 1: in perspektivischer Darstellung ein erstes Ausführungsbeispiel eines erfindungsgemässen autonomen Herzkreislauf-Implantats mit einem, einen blasenartig ausdehnbaren Bereich aufweisenden, stromaufwärts liegenden Leitungsabschnitt neben einem stromabwärts liegenden Leitungsabschnitt und einem Rückschlagventil, wobei der stromabwärts liegende Leitungsabschnitt mit dem stromaufwärts liegenden Leitungsabschnitt durch einen u-förmigen Verbindungsleitungsabschnitt strömungsverbunden ist und die Leitungsabschnitte in einem, hier als durchsichtig dargestellten, Gehäuse geführt sind;

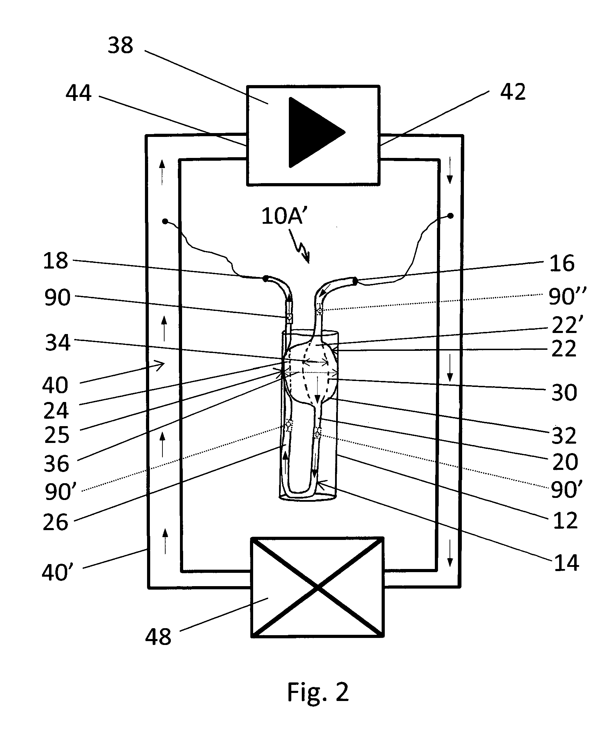

- Fig. 2: ein Schema eines eine pulsierende Quelle und eine Last aufweisenden Fluid-Kreislaufsystems mit einer autonomen Vorrichtung gemäss Fig. 1, welche als Bypass zur Last geschaltet ist;

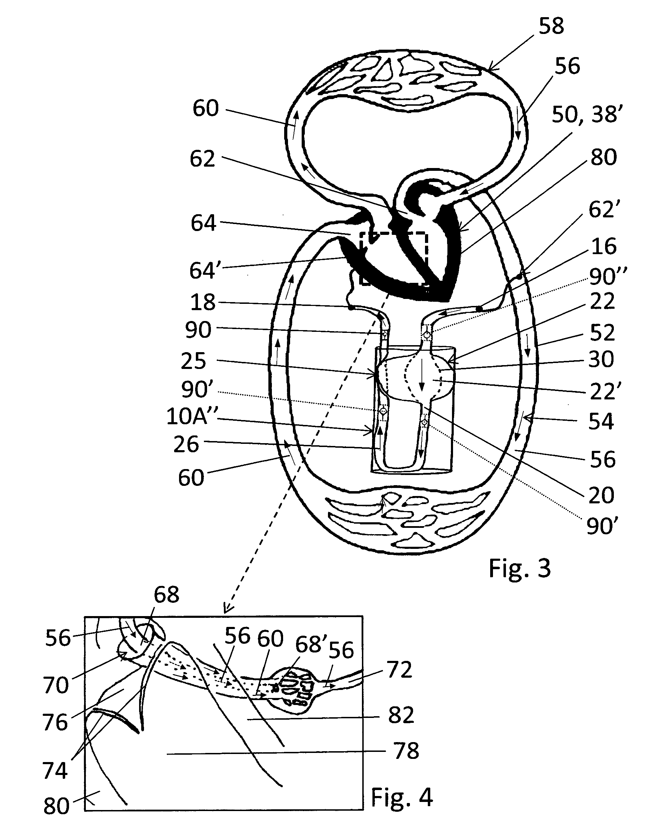

- Fig. 3: das menschliche Blut-Kreislaufsystem und ein entsprechend Fig. 1 ausgebildetes erfindungsgemässes autonomes Herzkreislauf-Implantat mit Rückschlagventil zur Unterstützung eines ischämischen Herzens;
- Fig. 4: eine detaillierte Darstellung eines in Fig. 3 gezeigten Ausschnitts mit einer Führung eines Katheters vom Koronarvenensinus zu den Herzkranzarterien;

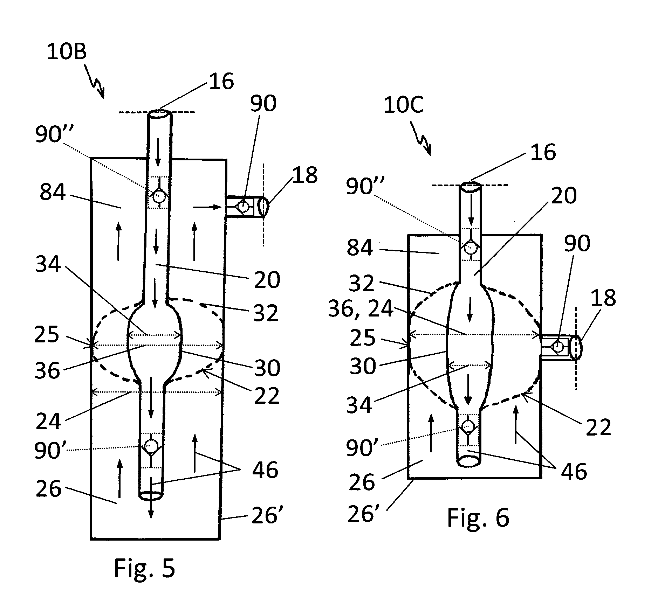

- Fig. 5: einen Querschnitt durch ein zweites Ausführungsbeispiel eines erfindungsgemässen autonomen Herzkreislauf-Implantats mit dem, den blasenartig ausdehnbaren Bereich aufweisenden, stromaufwärts liegenden Leitungsabschnitt im Inneren des stromabwärts liegenden Leitungsabschnitts und einem Rückschlagventil, wobei der stromabwärts liegende Leitungsabschnitt ein Gehäuse ausbildet und der blasenartig ausdehnbare Bereich stromaufwärts eines niederdruckseitigen Ausgangs der vorrichtung angeordnet ist; und
- Fig. 6: einen Querschnitt durch ein drittes Ausführungsbeispiel eines erfindungsgemässen autonomen Herzkreislauf-Implantats mit dem, den blasenartig ausdehnbaren Bereich aufweisenden, stromaufwärts liegenden Leitungsabschnitt im Inneren des stromabwärts liegenden Leitungsabschnitts und einem Rückschlagventil, wobei der stromabwärts liegende Leitungsabschnitt ein Gehäuse ausbildet und der blasenartig ausdehnbare Bereich neben dem niederdruckseitigen Ausgang der Vorrichtung angeordnet ist.

Eine in Fig. 1 gezeigte Vorrichtung 10A, insbesondere ein autonomes Herzkreislauf-Implantat 10A'', gemäss eines ersten Ausführungsbeispiels der vorliegenden Erfindung weist ein zylinderförmiges Gehäuse 12 mit einem darin u-förmig angeordneten schlauchförmigen Leitungssystem 14 auf.

Das Leitungssystem 14 hat einen hochdruckseitigen Eingang 16 und einen niederdruckseitigen Ausgang 18. Der hochdruckseitige Eingang 16 und der niederdruckseitige Ausgang 18 sind in vorliegendem Beispiel ausserhalb des Gehäuses 12 angeordnet.

Es ist jedoch auch denkbar, den hochdruckseitigen Eingang 16 und den niederdruckseitigen Ausgang 18 am Gehäuse 12 anzubringen.

Vom hochdruckseitigen Eingang 16 - in Strömungsrichtung-weggehend weist das Leitungssystem 14 einen stromaufwärts liegenden Leitungsabschnitt 20 mit einem blasenartig ausdehnbaren Bereich 22 auf. Dieser verläuft wenigstens annährend geradlinig und im Wesentlichen parallel zur Längsrichtung des Gehäuses 12 und in dessen Innenraum.

Zudem weist das Leitungssystem 14 zum niederdruckseitigen Ausgang 18 führend einen, einen Strömungsquerschnitt 24 aufweisenden, stromabwärts liegenden Leitungsabschnitt 26 auf. Dieser verläuft im Innenraum des Gehäuses wenigstens annährend parallel zur Längsrichtung und entlang der Wand 12' des Gehäuses 12.

Der stromaufwärts liegende Leitungsabschnitt 20 ist durch einen u-förmigen Verbindungsleitungsabschnitt 28 mit dem stromabwärts liegenden Leitungsabschnitt 26 strömungsverbunden. Hierbei ist der stromaufwärts liegende Leitungsabschnitt 20 mit seinem blasenartig ausdehnbaren Bereich 22 ausserhalb jedoch neben dem stromabwärts liegenden Leitungsabschnitt 26 im Gehäuse 12 angeordnet, so dass ein Strömungspfad 46 vom hochdruckseitigen Eingang 16 zum niederdruckseitigen Ausgang 18 ausgebildet ist.

Das Leitungssystem 14 ist abzweiglos vom hochdruckseitigen Eingang 16 zum niederdruckseitigen Ausgang 18 ausgebildet.

Das Gehäuse 12 ist vorzugsweise aus biokompatiblem Material wie Karbon oder Titan hergestellt. Auch das Material des Leitungssystems 14 ist biokompatibel, damit der Körper das Herzkreislauf-Implantat 10A' nicht als Fremdkörper abstösst.

Die Innenwand 14' des Leitungssystems 14 ist vorzugsweise mit einer glatten Oberfläche und mit einer gerinnungsfreien Beschichtung, insbesondere Heparinbeschichtung, versehen. Auf diese Weise kann die Gefahr einer Gerinnung erheblich reduziert werden und der Strömungspfad 46 fliesst ausschliesslich laminar durch das Leitungssystem 14.

In Fig. 1 ist gestrichelt ein erschlaffter Zustand 30 des blasenartig ausdehnbaren Bereichs 22 und durchgezogen ein gedehnter Zustand 32 des blasenartig ausdehnbaren Bereichs 22 dargestellt. Entsprechend ist der blasenartig ausdehnbare Bereich 22 elastisch zwischen dem erschlafften Zustand 30 mit einem Ruhequerschnitt 34 und dem gedehnten Zustand 32 mit einem bezüglich des Ruhequerschnitts 34 grösseren Arbeitsquerschnitts 36 hin und her bewegbar.

Im erschlafften Zustand 30 ist der stromabwärts liegende Leitungsabschnitt 26 mit seinem offenen Strömungsquerschnitt 24 und der stromaufwärts liegende Leitungsabschnitt 20 mit seinem Ruhequerschnitt 34 über die gesamte Gehäuselänge wenigstens annährend parallel zur Wand 12' des Gehäuses 12 geführt.

Im gedehnten Zustand 32 liegen einerseits der blasenartig ausdehnbare Bereich 22 und andrerseits der stromabwärts liegende Leitungsabschnitt 26 an der Wand 12' des Gehäuses 12 an. Hierbei nimmt der blasenartig ausdehnbare Bereich 22 im Innenraum des Gehäuses 12 den Arbeitsquerschnitt 36 ein und quetscht dabei den stromabwärts liegenden Leitungsabschnitt 26 in einem Reduktionsbereich 25.

Erfindungsgemäss ist im Strömungspfad 46 ein Rückschlagventil 90, 90', 90'' angeordnet, welches die Strömung vom hochdruckseitigen Eingang 16 zum niederdruckseitigen Ausgang 18 zulässt und in Gegenrichtung unterbindet.

Vorzugsweise ist das Rückschlagventil 90 stromabwärts des Reduktionsbereichs 25 angeordnet.

Optional ist das Rückschlagventil 90' zwischen dem blasenartig ausdehnbaren Bereich 22 und dem Reduktionsbereich 25 angeordnet.

Es ist auch denkbar, dass das Rückschlagventil 90'' zwischen dem hochdruckseitigen Eingang 16 und dem blasenartig ausdehnbaren Bereich 22 angeordnet ist.

Bei Verwendung der Vorrichtung 10A in einem eine pulsierende Quelle 38 aufweisenden Fluid-Kreislaufsystem 40, wie im Zusammenhang mit Fig. 2 im Detail erläutert, ist der hochdruckseitige Eingang 16 der Vorrichtung 10A dazu bestimmt, mit einem hochdruckseitigen Ausgang 42 der pulsierenden Quelle 38 strömungsverbunden zu sein. Zudem ist der niederdruckseitige Ausgang 18 der Vorrichtung 10A dazu bestimmt, mit einem niederdruckseitigen Eingang 44 der pulsierenden Quelle 38 strömungsverbunden zu sein.

Entsprechend ist im erschlafften Zustand 30 (gestrichelte Linie in Fig. 1) des blasenartig ausdehnbaren Bereichs 22 der Strömungsquerschnitt 24 des stromabwärts liegenden Leitungsabschnitts 26 freigegeben und im gedehnten Zustand 32 (durchgezogene Linie in Fig. 1) des blasenartig ausdehnbaren Bereichs 22 ist der Strömungsquerschnitt 24 des stromabwärts liegenden Leitungsabschnitts 26 im Reduktionsbereich 25, in vorliegendem gezeigten Fall auf Null, reduziert.

Das in Fig. 2 gezeigte Fluid-Kreislaufsystem 40 ist als rohrförmiges Leitungssystem 40' ausgebildet und umfasst die in Serie mit einer Last 48 geschaltete pulsierende Quelle 38.

Ein Fluid zirkuliert im Fluid-Kreinlaufsystem 40 vom hochdruckseitigen Ausgang 42 der pulsierenden Quelle 38 im Leitungssystem 40' durch die Last 48 zurück zum niederdruckseitigen Eingang 44 der pulsierenden Quelle 38. Entsprechend kommt es bei der Last 48 zu einem Druckabfall.

Bei einem bevorzugten Ausführungsbeispiel der vorliegenden Erfindung, wie in Fig. 2 gezeigt, ist die Vorrichtung 10A gemäss Fig. 1 als Bypass 10A' ausgeführt und parallel zur Last 48 des Fluid-Kreislaufsystems 40 angeordnet. Auf diese Weise fliesst ein grosser Teil des Fluids über die Last 48 und ein kleiner Teil des Fluids fliesst über den Bypass 10A' zum niederdruckseitigen Eingang 44 der pulsierenden Quelle 38 zurück.

Der Einbau der Vorrichtung 10A beziehungsweise des Bypass 10A' im Fluid-Kreislaufsystem 40 erfolgt derart, dass der hochdruckseitige Ausgang 42 der pulsierenden Quelle 38 mit dem hochdruckseitigen Eingang 16 der Vorrichtung 10A beziehungsweise des Bypass 10A' strömungsverbunden und der niederdruckseitige Eingang 44 der pulsierenden Quelle 38 mit dem niederdruckseitigen Ausgang 18 der Vorrichtung 10A beziehungsweise des Bypass 10A' strömungsverbunden ist.

Bei einem stossartigen Auswurf des Fluids, hervorgerufen durch die pulsierende Quelle 38, in das Leitungssystem 40' nimmt der blasenartig ausdehnbare Bereich 22 des Bypass 10A' im stromaufwärts liegenden Leitungsabschnitt 20 das Fluid von der pulsierenden Quelle 38 auf und geht vom Ruhequerschnitt 34 in den bezüglich des Ruhequerschnitts 34 grösseren Arbeitsquerschnitt 36 über. Auf diese Weise wirkt der blasenartig ausdehnbare Bereich 22 mit dem Gehäuse 12 derart zusammen, dass der Strömungsquerschnitt 24 des stromabwärts liegenden Leitungsabschnitts 26 in einem Reduktionsbereich 25, im vorliegenden Fall, auf Null reduziert wird.

Im Strömungspfad 46 ist das Rückschlagventil 90, 90', 90'' angeordnet, welches die Strömung vom hochdruckseitigen Eingang 16 zum niederdruckseitigen Ausgang 18 zulässt und in Gegenrichtung unterbindet.

Vorzugsweise ist das Rückschlagventil 90 stromabwärts des Reduktionsbereichs 25 angeordnet.

Optional ist das Rückschlagventil 90' zwischen dem blasenartig ausdehnbaren Bereich 22 und dem Reduktionsbereich 25 angeordnet.

Es ist auch denkbar, dass das Rückschlagventil 90'' zwischen dem hochdruckseitigen Eingang 16 und dem blasenartig ausdehnbaren Bereich 22 angeordnet ist.

In bevorzugter Weise ist das Gehäuse 12 starr ausgebildet, um eine Gegenkraft zum gedehnten Zustand des blasenartig ausdehnbaren Bereichs 22 zu bilden, so dass dieser den stromabwärts liegenden Leitungsabschnitt 26 im Reduktionsbereich 25, im vorliegenden Beispiel, vollständig quetscht.

Entsprechend sammelt sich während des Druckstosses das von der pulsierenden Quelle 38 zugeführte Fluid im Inneren 22' des blasenartig ausdehnbaren Bereichs 22 des stromaufwärts liegenden Leitungsabschnitts 20 an und wird dort gespeichert, solange der Druckanstieg im Fluid-Kreislaufsystem 40 vorherrscht.

Sobald jedoch die pulsierende Quelle 38 im Fluid-Kreislaufsystem 40 einen Druckabfall hervorruft, gelangt der blasenartig ausdehnbare Bereich 22 vom gedehnten Zustand wieder in den erschlafften zustand 30 über und gibt den Strömungsquerschnitt 24 des stromabwärts liegenden Leitungsabschnitts 26 wieder frei.

Auf diese Weise kann das im Inneren 22' des blasenartig ausdehnbaren Bereichs 22 vorliegende Fluid in Richtung des niederdruckseitigen Ausgangs 18 der pulsierenden Quelle 38 fliessen, ohne zuvor über die Last 48 zu fliessen.

Dadurch ist es mit dem Bypass 10A' möglich, nach dem Prinzip der sogenannten Gegenpulsation, das im Inneren 22' des blasenartig ausdehnbaren Bereich 22 gespeicherte Fluid während des Druckstosses zu sammeln, welches dann bei absinkenden bzw. abgesunkenen Druck im Fluid-Kreislaufsystem 40 zielgerichtet genutzt werden kann.

Bei einem besonders bevorzugten Ausführungsbeispiel der vorliegenden Erfindung, wie in Fig. 3 schematisch gezeigt, ist die Vorrichtung 10A gemäss Fig. 1 als menschliches Herzkreislauf-Implantat 10A'' ausgeführt.

Das in Fig. 3 gezeigte Fluid-Kreislaufsystem 40' ist ein Herz-Kreislaufsystem 52 des menschlichen Herzens 50, wobei die pulsierende Quelle 38 gemäss Fig. 2 in vorliegendem Fall das Herz 50 ist.

Das Herz-Kreislaufsystem 52 hat einen grossen Körper-Kreislauf 54 zur Versorgung der Körperorgane (nicht gezeigt) mit sauerstoffreichem Blut 56 und einen kleinen Lungen-Kreislauf 58 zur Anreicherung des zurückgeführten sauerstoffarmen Blutes 60 aus den Körperorganen wieder mit Sauerstoff in einer Lunge (nicht gezeigt).

Da die Körperorgane das sauerstoffreiche Blut 56 für die entsprechende Funktionalität nutzen, kommt es dort entsprechenden zu einem Druckabfall im Herz-Kreislaufsystem 52, was mit der Last 48 aus Fig. 2 vergleichbar ist.

Während der systolischen Phase des Herzens 50, ist der hochdruckseitiger Ausgang 62 des Herzens 50 verschlossen, so dass sich ein entsprechender systolischer Druck im Herzen 50 aufbauen kann, der bei einem gesunden Menschen zwischen 100 mmHg und 130 mmHg liegt.

Sobald sich der hochdruckseitige Ausgang 62 des Herzens 50 öffnet, wird das sauerstoffreiche Blut 56 stossartig in Richtung der Körperorgane abgegeben, wobei dieser Vorgang als diastolische Phase des Herzens 50 bezeichnet wird, so dass im Blut-Kreislaufsystem 52 ein diastolischer Druck von zwischen 60 mmHg und 85 mmHg vorherrscht.

Die Verwendung des Herzkreislauf-Implantats 10A'' im Blut-Kreislaufsystem 52 erfolgt derart, dass der hochdruckseitige Eingang 16 des Herzkreislauf-Implantats 10A'' über eine sogenannte Punktion 62' des hochdruckseitigen Ausgangs 62 des Herzens 50, insbesondere einer grosskalibrigen Arterie, wie beispielsweise einer Halsschlagader (Arteria carotis) oder einer Unterschlüsselbeinarterie (Arteria subclavia), strömungsverbunden ist, und der niederdruckseitige Ausgang 18 des Herzkreislauf-Implantats 10A'' ist ebenfalls über eine weitere Punktion 64' des niederdruckseitigen Eingangs 64 des Herzens 50, insbesondere einer Herzmuskelvene 66 strömungsverbunden.

Erfindungsgemäss ist im Strömungspfad 46 das Rückschlagventil 90, 90', 90'' angeordnet, welches die Strömung vom hochdruckseitigen Eingang 16 zum niederdruckseitigen Ausgang 18 zulässt und in Gegenrichtung unterbindet.

Vorzugsweise ist das Rückschlagventil 90 stromabwärts des Reduktionsbereichs 25 angeordnet.

Optional ist das Rückschlagventil 90' zwischen dem blasenartig ausdehnbaren Bereich 22 und dem Reduktionsbereich 25 angeordnet.

Es ist auch denkbar, dass das Rückschlagventil 90'' zwischen dem hochdruckseitigen Eingang 16 und dem blasenartig ausdehnbaren Bereich 22 angeordnet ist.

Wie in Fig. 4 rein schematisch dargestellt, wird nach weiterer Punktion 64' der Herzmuskelvene 66, ein Katheter 68 durch die Herzmuskelvene 66 über einen Koronarvenensinus 70 (Sinus coronarius) bis zu den Herzkranzarterien 72 geführt, so dass das Ende 68' des Katheters 68 sich direkt bei den Herzkranzarterien 72 befindet. Der mit dem Ausgang 18, vorzugsweise abzweigungslos, strömungsverbundene Katheter 68 transportiert das sauerstoffreiche (arterielle) Blut 56 vom Herzkreislauf-Implantat 10A'' und ist gestrichelt angedeutet. Überdies sind zur besseren Orientierung in Fig. 4 die Herzklappen 74 zwischen dem rechten vorhof 76 und der rechten Herzkammer 78, der Herzmuskel 80, die Herzscheidewand 82 (Septum), sowie das sauerstoffarme (venöse) Blut 60 und das sauerstoffreiche (arterielle) Blut 56 der Herzkranzgefässe kenntlich gemacht.

Durch den strömungsverbundenen Anschluss des niederdruckseitigen Ausgangs 18 des Herzkreislauf-Implantats 10A'' mit den Herzkranzarterien 72 ermöglicht das Herzkreislauf-Implantat 10A'' eine Unterstützung des Herzmuskels 80 durch die zusätzliche arterielle Versorgung der Herzkranzarterien 72 während der diastolischen Phase, da das angestaute Blutvolumen im blasenartig ausdehnbaren Bereich 22 des stromaufwärts liegenden Leitungsabschnitts 20 unmittelbar genutzt werden kann, sobald der Strömungspfad 46 im stromabwärts liegenden Leitungsabschnitt 26 beim Druckabfall, also während der diastolischen Phase, wieder freigeben ist.

Entsprechend findet das Herzkreislauf-Implantat 10A'' Verwendung als Herzunterstützungssystem, insbesondere beim ischämischen Herzen 50. Unter dem ischämischen Herzen 50 wird hierbei die mangelnde Blutversorgung des Herzmuskels 80 während der diastolischen Phase verstanden, was wiederum zu einem geringeren Druckanstieg im Herzen 50 während der systolischen Phase führt, da der Herzmuskel 80 nicht mehr die gewünschte Leistung aufbringen kann.

Somit kann über die sogenannte retrograde Koronarperfusion während der diastolischen Phase mittels des Herzkreislauf-Implantats 10A'' die Durchblutung des Herzmuskels 80 erheblich verbessert werden. Unter der retrograden Koronarperfusion wird hierbei verstanden, dass das sauerstoffreiche Blut 56 retrograd, also rückläufig unmittelbar wieder über die Herzmuskelvene 66 in den Herzmuskel 80 geführt wird, ohne zuvor durch die Körperorgane zu fliessen.

Folglich wirkt das Herzkreislauf-Implantat 10A'' als Bypass für den grossen Körper-Kreislauf 54 nach dem Prinzip der Gegenpulsation.

Zwei weitere Ausführungsbeispiele von erfindungsgemässen Herzkreislaur-Implantaten sind in Fig. 5 (zweites Ausführungsbeispiel) und Fig. 6 (drittes Ausführungsbeispiel) gezeigt.

Das zweite und dritte Ausführungsbeispiel funktionieren nicht nach dem Prinzip der Gegenpulsation, wie im ersten Ausführungsbeispiel gemäss Fig. 1 bis Fig. 3, sondern nach einem Prinzip der Volumenverdrängung, welches im Folgenden genauer erläutert wird.

Fig. 5 zeigt (es werden bei Übereinstimmung die gleichen Begriffe wie im Zusammenhang mit dem ersten Ausführungsbeispiel gemäss Fig. 1 verwendet) ein Herzkreislauf-Implantat 10B mit dem hochdruckseitigen Eingang 16 und dem niederdruckseitigen Ausgang 18.

Der blasenartig ausdehnbare Bereich 22 des stromaufwärts liegenden Leitungsabschnitte 20 ist in einem Inneren 84 des stromabwärts liegenden Leitungsabschnitts 26 angeordnet.

Der stromabwärts liegende Leitungsabschnitt 26 ist als starres Gehäuse 26' ausbildet.

Im vorliegenden dritten Ausführungsbeispiel ist der niederdruckseitige Ausgang 18 im oberen Drittel des Gehäuses 26' angeordnet und der blasenartig ausdehnbare Bereich 22 des stromaufwärts liegenden Leitungsabschnitts 20 liegt unterhalb des niederdruckseitigen Ausgangs 18 vor.

Der stromaufwärts liegende Leitungsabschnitt 20 hat im stromabwärts liegenden Leitungsabschnitt 26 ein offenes Ende, so dass der Strömungspfad 46 des Fluids von der pulsierenden Quelle 38 ungehindert in den stromabwärts liegenden Leitungsabschnitt 26 fliessen und von dort am niederdruckseitigen Ausgang 18 austreten kann.

Bei Verwendung der Vorrichtung 10B im die pulsierende Quelle 38 aufweisenden Fluid-Kreislaufsystem 40, ist der hochdruckseitige Eingang 16 der Vorrichtung 10B dazu bestimmt, mit dem hochdruckseitigen Ausgang 42 der pulsierenden Quelle 38 strömungsverbunden zu sein, und der niederdruckseitige Ausgang 18 der Vorrichtung 10B ist dazu bestimmt, mit dem niederdruckseitigen Eingang 44 der pulsierenden Quelle 38 strömungsverbunden zu sein.

Gemäss Fig. 5 ist erkennbar, dass im erschlafften Zustand 30 (durchgezogene Linie) des blasenartig ausdehnbaren Bereichs 22 ein ringartiger Strömungsquerschnitt 24 des stromabwärts liegenden Leitungsabschnitts 26 freigegeben ist, und im gedehnten Zustand 32 (gestrichelte Linie) des blasenartig ausdehnbaren Bereichs 22 ist der ringartige Strömungsquerschnitt 24 des stromabwärts liegenden Leitungsabschnitts 26 in einem Reduktionsbereich 25, im vorliegenden Fall, auf Null reduziert.

Erfindungsgemäss ist im Strömungspfad 46 das Rückschlagventil 90, 90', 90'' angeordnet, welches die Strömung vom hochdruckseitigen Eingang 16 zum niederdruckseitigen Ausgang 18 zulässt und in Gegenrichtung unterbindet.

Vorzugsweise ist das Rückschlagventil 90 stromabwärts des Reduktionsbereichs 25 angeordnet.

Optional ist das Rückschlagventil 90' zwischen dem blasenartig ausdehnbaren Bereich 22 und dem Reduktionsbereich 25 angeordnet.

Es ist auch denkbar, dass das Rückschlagventil 90'' zwischen dem hochdruckseitigen Eingang 16 und dem blasenartig ausdehnbaren Bereich 22 angeordnet ist.

Bei vorliegender Druckwelle bzw. beim Druckanstieg durch die pulsierende Quelle 38 nimmt der blasenartig ausdehnbare Bereich 22 im stromaufwärts liegenden Leitungsabschnitt 20 das Fluid von der pulsierenden Quelle 38 auf und geht vom Ruhequerschnitt 34 in den bezüglich des Ruhequerschnitts 34 grösseren Arbeitsquerschnitt 36 über.

Im gedehnten Zustand 32 des blasenartig ausdehnbaren Bereichs 22 wirkt dieser entsprechend mit dem starren Gehäuse 26' derart zusammen, dass der Strömungsquerschnitt 24 des stromabwärts liegenden Leitungsquerschuitts 26 im Reduktionsbereich 25, in vorliegendem Fall, auf Null reduziert ist.

Das Gehäuse 26' ist, wie oben ausgeführt, vorzugsweise starr ausgebildet und bildet so die Gegenkraft zum gedehnten Zustand des blasenartig ausdehnbaren Bereichs 22, so dass dieser unterhalb des niederdruckseitigen Ausgangs 18 der Vorrichtung 10B gegen das Gehäuse 26' drückt.

Auf diese Weise wird jenes Fluidvolumen im Inneren 84 des stromabwärts liegenden Leitungsabschnitts 26 während des Druckanstiegs aus dem niederdruckseitigen Ausgang 18 herausgedrückt, welches durch den gedehnten Zustand des blasenartig ausdehnbaren Bereich 22 des stromaufwärts liegenden Leitungsabschnitts 20 eingenommen wurde, und zwar nach dem Prinzip der Volumenverdrängung.

Damit ist zudem die Situation gegeben, dass der Strömungspfad 46 vom hochdruckseitigen Eingang 16 zum niederdruckseitigen Ausgang 18 unterbrochen ist.

Sobald nun die pulsierende Quelle 38 den Druckabfall im Fluid-Kreislaufsystem 40 hervorruft, gelangt der blasenartig ausdehnbare Bereich 22 wieder in den erschlafften Zustand 30 zurück, so dass der Strömungsquerschnitt 24 des stromaufwärts liegenden Leitungsabschnitts 26 wieder freigegeben ist.

Auf diese Weise kann das zuvor verdrängte Fluidvolumen im Inneren 84 des stromabwärts liegenden Leitungsabschnitts 26 wieder mit dem Fluid aus dem ausdehnbaren Bereich 22 aufgefüllt werden.

Somit wird beim zweiten Ausführungsbeispiel gemäss Fig. 5 das vom blasenartig ausdehnbaren Bereich 22 verdrängte Fluidvolumen während des Druckanstiegs oberhalb des blasenartig ausdehnbaren Bereichs 26 aus dem Inneren 84 des stromabwärts liegenden Leitungsabschnitts 26 in Richtung des niederdruckseitigen Ausgangs 18 der Vorrichtung 10B heraus gedrückt, während beim ersten Ausführungsbeispiel gemäss Fig. 1 bis 3 das angestaute Fluidvolumen im Inneren 22' des blasenartig ausdehnbaren Bereichs 22 beim Druckabfall genutzt wird.

Bei einem Herzkreislauf-Implantat 10C gemäss dem dritten Ausführungsbeispiel der vorliegenden Erfindung, wie in Fig. 6 gezeigt, liegt der gleiche Sachverhalt wie beim zweiten Ausführungsbeispiel gemäss Fig. 5 vor, jedoch wird im gedehnten Zustand 32 des blasenartig ausdehnbaren Bereiches 22 des stromaufwärts liegenden Leitungsabschnitts 20 der niederdruckseitige Ausgang 18 im stromabwärts liegenden Leitungsabschnitt 26 beim Druckanstieg vollständig verschlossen und beim Druckabfall wieder geöffnet.

Entsprechend findet auch hier eine Volumenverdrängung während des Druckanstiegs statt, wobei aus dem niederdruckseitigen Ausgang 18 der Vorrichtung 10C das verdrängte Fluidvolumen herausgedrückt wird.

An dieser Stelle sei erwähnt, dass es auch denkbar ist, dass sämtliche Ausführungsbeispiele der vorliegenden Erfindung miteinander frei kombinierbar sind, so dass beim Druckabfall gemäss dem ersten Ausführungsbeispiel ebenso wie beim Druckanstieg gemäss dem zweiten und dritten Ausführungsbeispiel das betreffende gestaute Fluidvolumen bzw. das verdrängte Fluidvolumen genutzt werden kann.

Überdies ist auch eine Verwendung der Vorrichtung 10B gemäss dem zweiten Ausführungsbeispiel bzw. der Vorrichtung 10C gemäss dem dritten Ausführungsbeispiel als Herzkreislauf-Implantat 10B' bzw. 10C' im BlutKreislaufsystem 52 gemäss Fig. 2 möglich.

Entsprechend könnte während der systolischen Phase, also beim Druckanstieg im Herzen 50, dass aus dem Inneren 68 des stromabwärts liegenden Leitungsabschnitts 26 verdrängte Blutvolumen zur zusätzlichen Versorgung des Herzmuskels 80 über die Herzkranzarterien 72 des Herzens 50 genutzt werden.

Bei einem weiteren nicht gezeigten Ausführungsbeispiel der vorliegenden Erfindung ist es denkbar, dass der Innenraum des Gehäuses 12 mit einem, gegebenenfalls unter Druck stehenden Gas gefüllt ist, welches den blasenartig ausdehnbaren Bereich 22 des stromaufwärts liegenden Leitungsabschnitts 20 im erschlafften Zustand 30 in einer bestimmten Ruheposition im Gehäuse 12 hält. Sobald nun ein Druckanstieg, hervorgerufen durch die pulsierende Quelle 38, den blasenartig ausdehnbaren Bereich 22 ausdehnt, muss dieser einer Gegenkraft des Gasdrucks entgegenwirken, um den Strömungsquerschnitt 24 im stromabwärts liegenden Leitungsabschnitt 26 zu reduzieren, und bei einem Druckabfall, hervorgerufen durch die pulsierende Quelle 38, komprimiert das Gas den blasenartig ausdehnbaren Bereich 22 wieder in Richtung der Ruheposition, so dass der Strömungsquerschnitt 24 wieder frei ist. Entsprechend kann der blasenartig ausdehnbare Bereich 22 mit keiner oder nur geringer Eigenelastizität ausgebildet werden (z.B. in der Form eines Balgs). Das Gas im Gehäuse 12 wirkt als Gasdruckfeder für den blasenartig ausdehnbaren Bereich 22.

Der Vollständigkeit halber sei erwähnt, dass der Querschnitt des Strömungspfads des Implantats, beziehungsweise der Vorrichtung, über seine gesamte Länge - mit Ausnahme im blasenartig ausdehnbaren Bereich - vorzugsweise konstant ist.

Die Erfindung betrifft weiter eine:
1. Autonome Vorrichtung für ein eine pulsierende Quelle (38) aufweisendes Fluid-Kreislaufsystem (40), wobei die pulsierende Quelle (38) einen hochdruckseitigen Ausgang (42) und einen niederdruckseitigen Eingang (44) aufweist, mit
   einem hochdruckseitigen Eingang (16), der dazu bestimmt ist mit dem hochdruckseitigen Ausgang (42) der pulsierenden Quelle (38) strömungsverbunden zu sein;
   einem niederdruckseitigen Ausgang (18), der dazu bestimmt ist mit dem niederdruckseitigen Eingang (44) der pulsierenden Quelle (38) strömungsverbunden zu sein;
   einem Strömungspfad (46) vom hochdruckseitigen Eingang (16) zum niederdruckseitigen Ausgang (18);
   einem vom hochdruckseitigen Eingang (16) weggehenden, stromaufwärts liegenden Leitungsabschnitt (20) des Strömungspfades (46) mit einem blasenartig ausdehnbaren Bereich (22) und
   einem zum niederdruckseitigen Ausgang (18) führenden, einen Strömungsquerschnitt (24) aufweisenden, stromabwärts liegenden Leitungsabschnitt (26) des Strömungspfades (46); wobei
   der stromaufwärts liegende Leitungsabschnitt (20) mit seinem blasenartig ausdehnbaren Bereich (22) entweder ausserhalb jedoch neben dem stromabwärts liegenden Leitungsabschnitt (26) oder im Inneren (84) des stromaufwärts liegenden Leitungsabschnitts (26) angeordnet ist;
   der blasenartig ausdehnbare Bereich (22) elastisch zwischen einem erschlafften Zustand (30) mit einem Ruhequerschnitt (34) und einem gedehnten Zustand (32) mit einem bezüglich des Ruhequerschnitts (34) grösseren Arbeitsquerschnitt (36) hin und her bewegbar ist; so dass
   der stromaufwärts liegende Leitungsabschnitt (20) und der stromabwärts liegende Leitungsabschnitt (26) derart miteinander zusammenwirken, dass
   bei einem durch die pulsierende Quelle (38) hervorgerufenen Druckanstieg am hochdruckseitigen Eingang (16) der blasenartig ausdehnbare Bereich (22) vom erschlafften Zustand (30) in den gedehnten Zustand (32) über geht und dabei den Strömungsquerschnitt (24) des stromabwärts liegenden Leitungsabschnitts (26) in einem Reduktionsbereich (25) reduziert; und
   bei einem durch die pulsierende Quelle (38) hervorgerufenen Druckabfall am hochdruckseitigen Eingang (16) der blasenartig ausdehnbare Bereich (22) vom gedehnten Zustand (32) in den erschlafften Zustand (30) über geht und dabei den Strömungsquerschnitt (24) des stromabwärts liegenden Leitungsabschnitts (26) freigibt; wobei
   im Strömungspfad (46) ein Rückschlagventil (90, 90', 90'') angeordnet ist, welches die Strömung vom hochdruckseitigen Eingang (16) zum niederdruckseitigen Ausgang (18) zulässt und in Gegenrichtung unterbindet.
2. Autonome Vorrichtung nach Anspruch 1, dadurch gekennzeichnet, dass das Rückschlagventil (90) stromabwärts des Reduktionsbereichs (25) angeordnet ist.
3. Autonome Vorrichtung nach Anspruch 1, dadurch gekennzeichnet, dass das Rückschlagventil (90') zwischen dem blasenartig ausdehnbaren Bereich (22) und dem Reduktionsbereich (25) angeordnet ist.
4. Autonome Vorrichtung nach einem der Ansprüche 1 bis 3, dadurch gekennzeichnet, dass bei vollständig gedehntem Zustand (32) des blasenartig ausdehnbaren Bereichs (22) der Strömungsquerschnitt (24) des stromabwärts liegenden Leitungsabschnitts (26) im Reduktionsbereich (25) auf Null reduziert ist.
5. Autonome Vorrichtung nach einem der Ansprüche 1 bis 4, dadurch gekennzeichnet, dass bei der Anordnung des blasenartig ausdehnbaren Bereichs (22) ausserhalb jedoch neben dem stromabwärts liegenden Leitungsabschnitt (26), dieser mit dem stromaufwärts liegenden Leitungsabschnitt (20) durch einen u-förmigen Verbindungsleitungsabschnitt (28) strömungsverbunden ist und die genannten Leitungsabschnitte (20, 26, 28) in einem Gehäuse (12) aufgenommen sind.
6. Autonome Vorrichtung nach einem der Ansprüche 1 bis 4, dadurch gekennzeichnet, dass bei der Anordnung des blasenartig ausdehnbaren Bereichs (22) im Inneren (84) des stromabwärts liegenden Leitungsabschnitts (26), letzterer als Gehäuse (26') ausbildet ist.
7. Autonome Vorrichtung nach einem der Ansprüche 1 bis 6, dadurch gekennzeichnet, dass der Strömungspfad (46) vom hochdruckseitigen Eingang (16) zum niederdruckseitigen Ausgang (18) abzweiglos ausgebildet ist.

Eine Aufgabe gemäss dieser Ausführungsform der Erfindung liegt darin, eine Vorrichtung zu schaffen, welche autonom - ohne eine externe Energiequelle und ohne eine externe Steuereinheit - mit einem eine pulsierende Quelle aufweisenden Fluid-Kreislaufsystem zusammenwirkt.

Bei dieser Ausführungsform ist eine autonome Vorrichtung für ein eine pulsierende Quelle aufweisendes Fluid-Kreislaufsystem vorgesehen, wobei die pulsierende Quelle einen hochdruckseitigen Ausgang und einen niederdruckseitigen Eingang aufweist. Die Vorrichtung hat einen hochdruckseitigen Eingang, der dazu bestimmt ist, mit dem hochdruckseitigen Ausgang der pulsierenden Quelle strömungsverbunden zu sein, und einen niederdruckseitigen Ausgang, der dazu bestimmt ist, mit dem niederdruckseitigen Eingang der pulsierenden Quelle strömungsverbunden zu sein. Entsprechend ist ein Strömungspfad vom hochdruckseitigen Eingang zum niederdruckseitigen Ausgangs ausgebildet. Zudem hat die Vorrichtung einen vom hochdruckseitigen Eingang weggehenden, stromaufwärts liegenden Leitungsabschnitt des Strömungspfades mit einem blasenartig ausdehnbaren Bereich und einen zum niederdruckseitigen Ausgang führenden, einen strömungsquerschnitt aufweisenden, stromabwärts liegenden Leitungsabschnitt des Strömungspfades. Der stromaufwärts liegende Leitungsabschnitt ist mit seinem blasenartig ausdehnbaren Bereich entweder ausserhalb jedoch neben dem stromabwärts liegenden Leitungsabschnitt oder im Inneren des stromaufwärts liegenden Leitungsabschnitts angeordnet. Der blasenartig ausdehnbare Bereich ist elastisch zwischen einem erschlafften Zustand mit einem Ruhequerschnitt und einem gedehnten Zustand mit einem bezüglich des Ruhequerschnitts grösseren Arbeitsquerschnitt hin und her bewegbar. Auf diese Weise wirken der stromaufwärts liegende Leitungsabschnitt und der stromabwärts liegende Leitungsabschnitt derart miteinander zusammen, dass bei durch die pulsierende Quelle hervorgerufenen Druckanstieg am hochdruckseitigen Eingang der blasenartig ausdehnbare Bereich vom erschlafften Zustand in den gedehnten Zustand über geht und dabei den Strömungsquerschnitt des stromabwärts liegenden Leitungsabschnitts in einem Reduktionsbereich reduziert und bei einem durch die pulsierende Quelle hervorgerufenen Druckabfall am hochdruckseitigen Eingang, der blasenartig ausdehnbare Bereich vom gedehnten Zustand in den erschlafften Zustand über geht und dabei den Strömungsquerschnitt des stromabwärts liegenden Leitungsabschnitts freigibt, wobei im Strömungspfad ein Rückschlagventil angeordnet ist, welches die Strömung vom hochdruckseitigen Eingang zum niederdruckseitigen Ausgang zulässt und in Gegenrichtung unterbindet.

Auf diese Art und Weise kann - im montierten Zustand der Vorrichtung - mittels des Rückschlagventils ein Rückfluss des Fluids vom niederdruckseitigen Eingang der pulsierenden Quelle in Richtung zum niederdruckseitigen Ausgang der Vorrichtung unterbunden werden.

Mit dem Begriff pulsierende Quelle ist gemeint, dass am hochdruckseitigen Ausgang der Quelle ein hoher Fluid-Anfangsdruck vorliegt und sobald das Fluid aus der Quelle in das Fluid-Kreislaufsystem strömt, fällt am hochdruckseitigen Ausgang der Quelle der Anfangsdruck ab. Der alternierende Wechsel zwischen dem hohen Fluiddruck und niedrigen Fluiddruck am Ausgang der Quelle in zeitlicher Abfolge wird als pulsierend verstanden.

Bei einer bevorzugten Ausführungsform der autonomen Vorrichtung ist das Rückschlagventil stromabwärts des Reduktionsbereichs angeordnet. Durch diese spezifische Anordnung des Rückschlagventils kann selbst bei reduziertem Leitungsquerschnitt des stromabwärts liegenden Leitungsabschnitts im Reduktionsbereich - im montierten Zustand der autonomen Vorrichtung - das Fluid immer nur vom hochdruckseitigen Eingang zum niederdruckseitigen Ausgang fliessen und nicht umgekehrt. Dies hat den vorteilhaften Effekt, dass mittels des Rückschlagventils eine Strömung des Blutes vom niederdruckseitigen Ausgang zum hochdruckseitigen Eingang des Herzkreislauf-Implantats vollständig unterbunden ist.

Bei einer bevorzugten Ausführungsform der autonomen Vorrichtung ist das Rückschlagventil zwischen dem blasenartig ausdehnbaren Bereich und dem Reduktionsbereich angeordnet. Diese spezifische Anordnung des Rückschlagventils hat den Vorteil, dass - im montierten Zustand der Vorrichtung während des Druckanstiegs der pulsierenden Quelle - das Fluid vom blasenartig ausdehnbaren Bereich ausschliesslich in Richtung zum Reduktionsbereich abfliessen kann.

Es ist auch denkbar, dass in einer Ausführungsform der autonomen Vorrichtung das Rückschlagventil zwischen dem hochdruckseitigen Eingang und dem blasenartig ausdehnbaren Bereich angeordnet ist. Dies hätte zur Folge, dass - im montierten Zustand der Vorrichtung während des Druckanstiegs der pulsierenden Quelle - das Fluid nicht über den hochdruckseitigen Eingang wieder zurück in das Fluid-Kreislaufsystem zurückfliessen kann.

Bei einer bevorzugten Ausführungsform der autonomen vorrichtung ist bei vollständig gedehntem Zustand des blasenartig ausdehnbaren Bereichs der Strömungsquerschnitt des stromabwärts liegenden Leitungsabschnitts im Reduktionsbereich auf Null reduziert.

Bei einer bevorzugten Ausführungsform der autonomen Vorrichtung ist bei der Anordnung des blasenartig ausdehnbaren Bereichs ausserhalb jedoch neben dem stromabwärts liegenden Leitungsabschnitt, dieser mit dem stromaufwärts liegenden Leitungsabschnitt durch einen u-förmigen Verbindungsleitungsabschnitt strömungsverbunden und die genannten Leitungsabschnitte sind in einem Gehäuse aufgenommen.

Bei einer bevorzugten Ausführungsform der autonomen Vorrichtung ist bei der Anordnung des blasenartig ausdehnbaren Bereichs im Inneren des stromabwärts liegenden Leitungsabschnitts, letzterer als Gehäuse ausbildet.

Bei einer bevorzugten Ausführungsform der autonomen Vorrichtung ist der Strömungspfad vom hochdruckseitigen Eingang zum niederdruckseitigen Ausgang abzweiglos ausgebildet.

Die Erfindung betrifft überdies:
1. Ein Fluid-Kreislaufsystem mit einer pulsierenden Quelle (38) und einer autonomen Vorrichtung (10A, 10B, 10C) nach einem der Ansprüche 1 bis 7, wobei das Fluid-Kreislaufsystem (40) kein Herzkreislaufsystem des menschlichen oder tierischen Körpers ist.

Bei dieser Ausführungsform weist das Fluid-Kreislaufsystem die pulsierende Quelle und die autonome Vorrichtung wie oben beschrieben auf, wobei das Fluid-Kreislaufsystem kein Herzkreislaufsystem des menschlichen oder tierischen Körpers ist.

Dieses Fluid-Kreislaufsystem hat die pulsierende Quelle, das in sich geschlossenes Leitungssystem und die im Leitungssystem angeordnete Last. Entsprechend existiert ein kontinuierlicher Strömungspfad von einem hochdruckseitigen Ausgang der Quelle über die Last zum niederdruckseitigen Eingang der Quelle zurück. Die im Leitungssystem angeordnete Last bewirkt im Fluid-Kreislaufsystem einen Druckabfall, wobei das Fluid als Pulswelle mit einem hohen Anfangsdruck von der pulsierenden Quelle in das Fluid-Kreislaufsystem abgegeben wird.

Überdies ist ein weitere Aspekt der vorliegenden Erfindung die Verwendung der oben beschriebenen autonomen Vorrichtung zur Unterstützung des Auswurfvolumens eines ischämischen Herzens in einem Blutkreislaufsystem, wobei das Herz die pulsierende Quelle mit einer Arterie als hochdruckseitigen Ausgang und einer Vene als niederdruckseitigen Eingang aufweist. Der hochdruckseitige Eingang der Vorrichtung ist bei einer derartigen Verwendung dazu bestimmt, mit der Arterie des Herzens strömungsverbunden zu sein, und der niederdruckseitige Ausgang der Vorrichtung ist bei dieser Verwendung dazu bestimmt, mit der Vene des Herzens strömungsverbunden zu sein. Entsprechend kann ein Blutstrom pulsatil vom hochdruckseitigen Eingang zum niederdruckseitigen Ausgang fliessen. Der blasenartig ausdehnbare Bereich der Vorrichtung ist zwischen dem erschlafften Zustand mit dem Ruhequerschnitt und dem gedehnten Zustand mit dem bezüglich des Ruhequerschnitts grösseren Arbeitsquerschnitt hin und her bewegbar, so dass der stromaufwärts liegende Leitungsabschnitt und der stromabwärts liegende Leitungsabschnitt derart miteinander zusammenwirken können, dass bei durch das Herz hervorgerufenen Druckanstieg, also in der systolischen Phase, am hochdruckseitigen Eingang, der blasenartig ausdehnbare Bereich vom erschlafften Zustand in den gedehnten Zustand übergeht und dabei den Strömungsquerschnitt des stromabwärts liegenden Leitungsabschnitts wenigstens annährend vollständig reduziert, und bei einem durch das Herz hervorgerufenen Druckabfall, also in der diastolischen Phase, am hochdruckseitigen Eingang, der blasenartig ausdehnbare Bereich vom gedehnten Zustand in den erschlafften Zustand übergeht und dabei den Strömungsquerschnitt des stromabwärts liegenden Leitungsabschnitts freigibt, wobei im Strömungspfad ein Rückschlagventil angeordnet ist, welches die Strömung vom hochdruckseitigen Eingang zum niederdruckseitigen Ausgang zulässt und in Gegenrichtung unterbindet.

Die Erfindung betrifft also die autonome Vorrichtung, insbesondere das Herzkreislauf-Implantat, für ein eine pulsierende Quelle Aufweisendes Fluidkreislaufsystem zur Unterstützung des Auswurfvolumens eines ischämischen Herzens in einem Blutkreislaufsystem.

## Patentansprüche

1. Autonomes Herzkreislauf-Implantat aufweisend:
einen hochdruckseitigen Eingang (16), der dazu bestimmt ist mit einer Arterie eines Herzkreislaufsystems (52) strömungsverbunden zu sein;
einen niederdruckseitigen Ausgang (18), der dazu bestimmt ist mit einer Vene, insbesondere einer Herzmuskelvene (66), des Herzkreislaufsystems (52) strömungsverbunden zu sein;
einen Strömungspfad (46) vom hochdruckseitigen Eingang (16) zum niederdruckseitigen Ausgang (18);
einen vom hochdruckseitigen Eingang (16) weggehenden, stromaufwärts liegenden Leitungsabschnitt (20) des Strömungspfades (46) mit einem blasenartig ausdehnbaren Bereich (22) und
einen zum niederdruckseitigen Ausgang (18) führenden, einen Strömungsquerschnitt (24) aufweisenden, stromabwärts liegenden Leitungsabschnitt (26) des Strömungspfades (46); wobei
der stromaufwärts liegende Leitungsabschnitt (20) mit seinem blasenartig ausdehnbaren Bereich (22) entweder ausserhalb jedoch neben dem stromabwärts liegenden Leitungsabschnitt (26) oder im Inneren (84) des stromabwärts liegenden Leitungsabschnitts (26) angeordnet ist;
der blasenartig ausdehnbare Bereich (22) elastisch zwischen einem erschlafften Zustand (30) mit einem Ruhequerschnitt (34) und einem gedehnten Zustand (32) mit einem bezüglich des Ruhequerschnitts (34) grösseren Arbeitsquerschnitt (36) hin und her bewegbar ist; so dass
im erschlafften Zustand (30) des blasenartig ausdehnbaren Bereichs (22) der Strömungsquerschnitt (24) des stromabwärts liegenden Leitungsabschnitts (26) freigegeben ist; und
im gedehnten Zustand (32) des blasenartig ausdehnbaren Bereichs (22) der Strömungsquerschnitt (24) des stromabwärts liegenden Leitungsabschnitts (26) in einem Reduktionsbereich (25) reduziert ist, und wobei
im Strömungspfad (46) ein Rückschlagventil (90, 90', 90'') angeordnet ist, welches die Strömung in Richtung vom hochdruckseitigen Eingang (16) zum niederdruckseitigen Ausgang (18) zulässt und in Gegenrichtung unterbindet.

2. Autonomes Herzkreislauf-Implantat nach Anspruch 1, **dadurch gekennzeichnet, dass** das Rückschlagventil (90) stromabwärts des Reduktionsbereichs (25) angeordnet ist.

3. Autonomes Herzkreislauf-Implantat nach Anspruch 1, **dadurch gekennzeichnet, dass** das Rückschlagventil (90') zwischen dem blasenartig ausdehnbaren Bereich (22) und dem Reduktionsbereich (25) angeordnet ist.

4. Autonomes Herzkreislauf-Implantat nach einem der Ansprüche 1 bis 3, **dadurch gekennzeichnet, dass** bei vollständig gedehntem Zustand (32) des blasenartig ausdehnbaren Bereichs (22) der Strömungsquerschnitt (24) des stromabwärts liegenden Leitungsabschnitts (26) auf Null reduziert ist.

5. Autonomes Herzkreirlauf-Implantat nach einem der Ansprüche 1 bis 4, **dadurch gekennzeichnet, dass** bei der Anordnung des blasenartig ausdehnbaren Bereichs (22) ausserhalb jedoch neben dem stromabwärts liegenden Leitungsabschnitt (26), dieser mit dem stromaufwärts liegenden Leitungsabschnitt (20) durch einen u-förmigen Verbindungsleitungsabschnitt (28) strömungsverbunden ist, und die genannten Leitungsabschnitte (20, 26, 28) in einem Gehäuse (12) aufgenommen sind.

6. Autonomes Herzkreislauf-Implantat nach einem der Ansprüche 1 bis 4, **dadurch gekennzeichnet, dass** bei der Anordnung des blasenartig ausdehnbaren Bereichs (22) im Inneren (84) des stromabwärts liegenden Leitungsabschnitts (26), letzterer als Gehäuse (26') ausbildet ist.

7. Autonomes Herzkreislauf-Implantat nach einem der Ansprüche 1 bis 6, **dadurch gekennzeichnet, dass** der Strömungspfad (46) vom hochdruckseitigen Eingang (16) zum niederdruckseitigen Ausgang (18) abzweiglos ausgebildet ist.
